# EUROPEAN PATENT APPLICATION

(11) **EP 1 881 076 A1**
(43) Date of publication of application: **23.01.2008**
(21) Application number: 06117294.6
(22) Date of filing: 17.07.2006
(51) Int. Cl.: C12P 1/04

(54) **Method for producing L-amino acids**

(71) Applicant: Evonik Degussa GmbH, 40474 Düsseldorf (DE)
(72) Inventor: Bathe, Brigitte, Dr., 33154 Saltzkotten (DE); Hans, Stephan, 49078 Osnabrück (DE); Claes, Wilfried, Dr., 33615 Bielefeld (DE)

(57) **Abstract**

The invention is directed to a process for producing a L-amino acid, comprising fermenting a microorganism, wherein the expression of a lysC gene encoding a feed back resistant aspartokinase is enhanced by cloning a strong promoter.

## Description

L-amino acids are used in the food sector, as pharmaceuticals and for animal nutrition. L-lysine is mainly used as additive for the preparation of feed.

The current world production for L-lysine is estimated to be more than 350 thousand tons a year. The production is based on fermentation by Corynebacterium glutamicum. Review articles concerning various aspects of L-amino acids can be found in volume 79 of Advances in Biochemical Engineering Biotechnology (volume editors: R. Faurie and J. Thommel) entitled "Microbial Production of L-Amino Acids".

The microbial L-lysine biosynthesis by Corynebacterium glutamicum is a well regulated process. Next to increasing the copy number of a gene the enhancement of enzyme activities can also be done by raising the expression value of a gene.

Recent patent applications demonstrate the use of various promoters for the overexpression of genes involved in L-amino acid biosyntheses or central metabolism (WO 2005/059144; WO 2005/059143; WO 2005/059093; WO 2006/008100; WO 2006/008101; WO 2006/008102; WO 2006/008103; WO 2006/008097; WO 2006/008098; US2006/0003424; WO 02/40679).

Now it was found that the expression of a lysC gene encoding a feed back resistant aspartokinase was enhanced significantly by cloning one of the polynucleotides described in table 1 in front of the lysC gene.

Each of the polynucleotides consists of a promoter sequence and a ribosome binding site. They can be obtained under the access number named in column B at the National Center for Biotechnology Information (NCBI, Bethesda, MD, USA). Under the specific accession number the applied sequence spans from position 1 (mentioned in column C) to position 2 (mentioned in column D) in table 1. The length of the polynucleotides is given in column E.

The sequences comprising the applied polynucleotides are termed seqP-RBS_01 to seqP-RBS_20 and shown in the sequence list.

The detailed sequence information is displayed in Fastaformat.

Each of the polynucleotides comprising the promoter and ribosome binding site (seqP-RBS) and the coding sequence of the lysC gene were cloned into plasmid pK18mobsacB (see WO03/014330 for experimental details). The exchange of the natural lysC promoter contained in the chromosome of the parent strain against seqP-RBS was achieved by conjugation and subsequent screening for homologous recombination events as described in WO03/014330. A strain was obtained which contained in its chromosome the seqP-RBS in front of the coding sequence of the lysC gene. Thus the expression of the aspartokinase was placed under the control of the seqP-RBS sequence.

Strains of Corynebacterium glutamicum carrying the combined seqP-RBS-lysC sequence produced significant higher amounts of L-lysine as compared to the parental strains. Enzyme activity analysis of these strains showed a higher aspartate kinase activity.

It was also found that significant overexpression of a number of genes known to be beneficial for L-lysine production (see WO03/014330, Table 1) can be achieved by placing the seqP-RBS as shown in the sequence list in front of said genes.

In particular expression of enzymes involved in the pentose phosphate pathway (e. g. glucose 6 phosphate dehydrogenase complex encoded by genes zwf and opcA) or involved in anaplerosis (e.g. phosphoenolpyruvate carboxylase encoded by the ppc gene and pyruvate carboxylase encoded by the pyc gene) were increased by cloning the seqP-RBS in front of said genes. The enhancement of the expression of these genes also resulted in a significant increase of L-lysine production.

**Table1:**

| A | B | C | D | E |
|---|---|---|---|---|
| no. | access number | position 1 | position 2 | length (bp) |
| 1 | BX927153.1 | 245335 | 245460 | 126 |
| 2 | BX927157.1 | 32411 | 32561 | 151 |
| 3 | BX927150.1 | 98920 | 99062 | 143 |
| 4 | BX927156.1 | 323078 | 323213 | 136 |
| 5 | BX927150.1 | 159092 | 159730 | 639 |
| 6 | BX927153.1 | 235993 | 236481 | 489 |
| 7 | BX927151.1 | 326822 | 327856 | 1035 |
| 8 | BX927156.1 | 20664 | 20797 | 134 |
| 9 | BX927155.1 | 201514 | 201977 | 464 |
| 10 | BX927155.1 | 245359 | 245603 | 245 |
| 11 | BX927150.1 | 228747 | 228933 | 187 |
| 12 | BX927148.1 | 274121 | 274323 | 203 |
| 13 | BX927151.1 | 120798 | 120921 | 124 |
| 14 | BX927155.1 | 137815 | 138019 | 205 |
| 15 | BX927152.1 | 125237 | 125375 | 139 |
| 16 | BX927157.1 | 15642 | 15769 | 128 |
| 17 | BX927154.1 | 207625 | 207866 | 242 |
| 18 | BX927156.1 | 134993 | 135239 | 247 |
| 19 | BX927154.1 | 256472 | 256676 | 205 |
| 20 | BX927152.1 | 289885 | 289402 | 484 |

## Claims

1. Process for producing an L-amino acid, comprising fermenting a microorganism, wherein the expression of a lysC gene encoding a feed back resistant aspartokinase is enhanced by cloning a promoter selected from the group consisting of seqP-RBS_01, seqP-RBS_02, seqP-RBS_03, seqP-RBS_04, seqP-RBS_05, seqP-RBS_06, seqP-RBS_07, seqP-RBS_08, seqP-RBS_09, seqP-RBS_10, seqP-RBS_11, seqP-RBS_12, seqP-RBS_13, seqP-RBS_14, seqP-RBS_15, seqP-RBS_16, seqP-RBS_17, seqP-RBS_18, seqP-RBS_19, seqP-RBS_20 in front of said lysC gene.


SEQUENCE LISTING

<110>  Degussa AG

<120>  Method for producing L-amino acids

<130>  200600371

<160>  20

<170>  PatentIn version 3.3

<210>  1
<211>  126
<212>  DNA
<213>  Corynebacterium glutamicum

<220>
<221>  misc_feature
<223>  seqP-RBS_01

<400>  1
ccagtcaatc ctaagagctt gaaacgcccc aatgtggggg tgttaagaac tccataaaag      60

cgcttgggaa ctttttgtgg aagcagtccg ttgaacctct tgaaccgcga atttaggagg     120

ccagtt                                                                126

<210>  2
<211>  151
<212>  DNA
<213>  Corynebacterium glutamicum

<220>
<221>  misc_feature
<223>  seqP-RBS_02

<400>  2
gacccctatt atagcactgc ttgataaatt gtcaccggcc cttagcctaa gcttatttat      60

attaggttgt cctatcctga aagtgccgat tcccgcaggc cacgcttagt tttttatcaa     120

atcggcacac cggctactaa ggttggtgat t                                    151

<210>  3
<211>  143
<212>  DNA
<213>  Corynebacterium glutamicum

<220>
<221>  misc_feature
<223>  seqP-RBS_03

<400>  3
tcttggatat aaaaggggcc cctattggca atgtggttat gaccagaagt agtataagaa      60

gtgaagttga tcgcgtcatc gtagtacgag taattccact cacacatcaa tgcggtgacc     120

acaattggga ggagaagtag cac                                             143

<210>  4

<211>  136
<212>  DNA
<213>  Corynebacterium glutamicum

<220>
<221>  misc_feature
<223>  seqP-RBS_04

<400>  4
gtcggtcgac ctttcattga ttggtgaatc ctgcgttaca gttcgtggaa actcatgatc      60

tctaacacct ctaagtgtag cgcggatacc cgatccgcac agctggttag taccagtatt     120

tttgaggctt ttgaca                                                     136

<210>  5
<211>  639
<212>  DNA
<213>  Corynebacterium glutamicum

<220>
<221>  misc_feature
<223>  seqP-RBS_05

<400>  5
atgaagagaa ataagccgac atttgatgtc ggctttttct tttttctcgg gctatttgag      60

cttgagtcgg gcggaaagcg cttcaacctg cggatttgta gaaaatctgg gagtaacgta     120

catttaaacg cacaccagca acgtggccaa gaggttacaa gctggttaag acatgcagaa     180

tgattaaaaa agaaactttg acttttttaa atcgactctg taaggtcaat caagctgcta     240

ggggagcaag tacaaacatt gtatcttgtt ttcagagtgt gcgtatgttg tttgagaact     300

caatagtgtg ccatttatta tttttgtcac taccacaacc acttaggttg tggtgggtca     360

tgccgggtgg tggatcgcca attttatcca tcactgtaaa caataatctt attgttggca     420

tgattttgtg ggtctgcttg ttccccgtca agggactagg cccacgttga agacacgatg     480

atccttatgt gggttgtggt gtttttttag aatcattatt ttttgataat gccagtaaca     540

ccccattatc tgatcgcact gtgtgtggtt ggtgggggtg ttgttgtttt tgtcaggtgt     600

tgggcttttc acggcctgaa caatctattt attgttttt                            639

<210>  6
<211>  489
<212>  DNA
<213>  Corynebacterium glutamicum

<220>
<221>  misc_feature
<223>  seqP-RBS_06

<400>  6
gttatcgaaa ggtgattttt gcccagggcc ttgattcaca acgcaccttg ctgtaggaaa      60

aacaggcccc tttgtgacat cggcgtagtt gttcaactat aatggaacgc tgatcgtgga     120

caagagttaa ccatgagatt gattcacccc tttaagcctc caaagaagta gttgactcaa     180

cgcatttcgg catttaaaaa agccgagagc aaatgagact ttccaggaga aggcaccagg 240

gacatgaaca attgatcggc tgaccaactc tataagagat gcacctcaag tttggggata 300

cttattcggc gtttctgggg aacaaatacg tttccctatt gttgtatata ggtattcgca 360

cttaagaaac atctctcatg gaaagaagct aggcggaaag ggcgttaagt acttgccatt 420

taatcctcag catcactcgg atcagtcgga gatgtcgatg aaaatgcacc aggagccgtg 480

gagagcagc 489

<210> 7
<211> 1035
<212> DNA
<213> Corynebacterium glutamicum

<220>
<221> misc_feature
<223> seqP-RBS_07

<400> 7
tgcaccattc ctctcttcct tcgccggtgc taaccggaca ggttcaaacg gtgctcgcgc 60

agcaatcagc gcgatctcag cccgtgctcg ggcgcccgtg gggactgcct tcaaaactag 120

cgcgttgcac gcgtgcgcgt aaaagttacc cccttgcggg ggttaagaga gggcgctttt 180

cgacgcggtg atggtggggt acgcctacgt agtggttcta actcggccgt tgcttgcgaa 240

tctcagacac ccgtgagtgg tcttgaatcc aattagtggc atctttctca atccaagagg 300

cgacattaaa aactgctgaa tcaaacatat tgaggttcct atcgtcgacg tctaaatgtg 360

acttgctgag cggtgataat tttcccagga gaatcgtttg ttttagttct tcttttggcg 420

gttgttagag cttttttgaa ctgttgagca aatctaaaat agctatcatc ttcaaaccag 480

aaggggccac gagcagatcg taattgggcg tcaaccctgt ttcttaaaag cacttctaca 540

tgttgtatat cttctaaata tgctttagat aactgggtat tccagatgta aaaaccttcg 600

ggattttcag aatttcggta tgtattcatt ctagggatgg agaaccactg ttcgagctct 660

gcgatagatg taggggagtt gccagtcacg tcgccaaggt cttacactta ggattcctaa 720

gccccggagt gatccctacc attttggtac gtcaccgggg ctcttgtttt ttcttcaagc 780

gctcgtctct gatgtcctga aagagtggct agggtatcgg tgaatccatc gacacaaaag 840

gattgggaca ttataaattc ggcttgcatc tagaccaagc tgtctgctaa tgtcgatttt 900

acgatcaaga agatcagccg caagcccttc ggcaggctga ctggcaaccg cgcatcgcgc 960

acggtgcccc cgaaggaaga tcggctctgt actcaaaaga atgcagagaa gagcgatttt 1020

ttgaaggaga ttccc 1035

<210> 8
<211> 134
<212> DNA
<213> Corynebacterium glutamicum

<220>
<221>  misc_feature
<223>  seqP-RBS_08

<400>  8
aatcttcatc acatggtgtg tgcagaaata tttttgctgg tctattgtgg cgaccgaggg      60

cctttgaagg ttcgacaaac tgtataaggc cttgaatctt gagaatttat tttgaggaag     120

caagaggaag tgtc                                                       134


<210>  9
<211>  464
<212>  DNA
<213>  Corynebacterium glutamicum


<220>
<221>  misc_feature
<223>  seqP-RBS_09

<400>  9
taggtgcgct cttcccttct acaaattttt ttgcttttcg acgccacaat gcagcgcgat      60

ttcagatcgc cagcttaccc caaccgaagc ccttcagaag acccgcccca gcctatcaat     120

tttggtcaag tcgcctagct ttagacctag ctttagagcg caatccccca ccccaaaacg     180

tgatcttagt cacttatttt ccattacgcc ccacagggtt cagtgtcaga ctaacgccac     240

aagaccatct ccatcagccc tcaacttcaa ccacttattg ccctaaatta tcgccactag     300

cacgtcattt agagaaaacc tttaatgtga aacaggtctc aaactgggcc attttcaagc     360

cttcaaaaag gtgggaaact tagccaatcc aaagcccaaa aatgcgggtt atgctgcgct     420

aacctatgct gacagccttg cggaagttgt gtacgttagg ggcc                      464


<210>  10
<211>  245
<212>  DNA
<213>  Corynebacterium glutamicum


<220>
<221>  misc_feature
<223>  seqP-RBS_10

<400>  10
actaacttga tcaccttgct cggtgtctcg taagcagctt gttcgacttt acccttatcg      60

ttaatcagaa agcaacttgc gttaattcct gaagaatttt gggagtctgt cagcgtttca     120

atttcccttg ctgtgaagct cggttcgttg cgctgactcg aattaatcta cacagcccct     180

ctgacaagca caaaccccca gctcaatgat ggtttcgctg ggggatgctt gatgggaagg     240

cttcg                                                                 245


<210>  11
<211>  187
<212>  DNA
<213>  Corynebacterium glutamicum

<220>
<221>  misc_feature
<223>  seqP-RBS_11

<400>  11
aacttacttt ctcctagccg cccacattca gttagaagac gcgccgcacc gggtgaccgg      60

ggcgcgagca tgccagctga cggggcgata acgaatattt acgacaacag caagggacaa     120

gtttacatac gttgccccaa aacacctaat cgcctattca atgcgggttt agatcgcccc     180

ggaagcc                                                               187


<210>  12
<211>  203
<212>  DNA
<213>  Corynebacterium glutamicum


<220>
<221>  misc_feature
<223>  seqP-RBS_12

<400>  12
aattgctcag aataccagtt cagaccgggg tcaccataaa ggtgtgtagg gggtgtcaaa      60

gtgggcaaaa agtatcatac ttttgtcttt ttgctggcag tttacgtaag ggctgattat     120

ttggtggcgc gaagtgacat tctgatgtag actatcaatc agacttaatc cataggtaac     180

cctcataaaa ggaaggaatg cta                                             203


<210>  13
<211>  124
<212>  DNA
<213>  Corynebacterium glutamicum


<220>
<221>  misc_feature
<223>  seqP-RBS_13

<400>  13
gcgttatagt ctagagcgag caggcgagat gtgaagtacc tacacgcatt aagtgcaaat      60

gaattcacaa ttgccagaag atgcacagga tgtaatctag atttcccaag ttcagtgggg     120

caaa                                                                  124


<210>  14
<211>  205
<212>  DNA
<213>  Corynebacterium glutamicum


<220>
<221>  misc_feature
<223>  seqP-RBS_14

<400>  14
tgcagaatct cctccatcgg caggtacgtg ccgaacgatc cgtagtgtga gaaacagtga      60

agtgagtcac tgtgcgccat ctagagtgct gcctatgaca ttagcactag tttggaccct     120

atgacaaagc ccggaagcca tgccgtggct tccgggctta gttcttctaa ttcatgggcg     180

tggatgagac gccgcgggca tcgag                                           205

<210>  15
<211>  139
<212>  DNA
<213>  Corynebacterium glutamicum

<220>
<221>  misc_feature
<223>  seqP-RBS_15

<400>  15
cctgagaact atgaaattcc agctcacggc gttttttcag gaaattctag gatcttacgg      60

agggtaactc cgcactcaac atctagacaa aggctatgat gaaaatcgtt aagaataatg     120

tcgacacgga ggaagttta                                                  139

<210>  16
<211>  128
<212>  DNA
<213>  Corynebacterium glutamicum

<220>
<221>  misc_feature
<223>  seqP-RBS_16

<400>  16
ggacctcatc acctcctatg gtctagtaat tgttttttcg gccatgccgt cgttggcata      60

gcaggagggt cgttgcgtca gcaacctatt caaggtacac caaacgcgcg gaaatgtcga     120

ttcctgat                                                              128

<210>  17
<211>  242
<212>  DNA
<213>  Corynebacterium glutamicum

<220>
<221>  misc_feature
<223>  seqP-RBS_17

<400>  17
cgctttagtc tccttaactg ttggcccttt gaattacttt taggccggga catcataggc      60

ttgcagtgta ctcccctttt tacggatctc cggcgagtga tgctggatta cgttcatatg     120

ggaagcggat gggtgttccc cagcctactc accgtccaca gatgagtaaa cccggaaaaa     180

cccgtattta gttattggtt ttacctgcgt gggctgaaag tcttcacttt taatccttac     240

ag                                                                    242

<210>  18
<211>  247

<212>  DNA
<213>  Corynebacterium glutamicum


<220>
<221>  misc_feature
<223>  seqP-RBS_18

<400>  18
ccccgatagt gtatgtgctg acctgcgttt atgcattttc cttaggttga aggaaatatc      60

acacgacaaa agttgagtga tgcaggcata attggctata ggcaactgaa gatgccacaa     120

tcaatgtttg atccaacaga atcagacatt gtgaatgtgg gattatcggt tcgcgcttca     180

ccatgtttct gcatgatgaa attacataca tagttcagtg acagtcacct tttggaggag     240

acacctt                                                               247


<210>  19
<211>  205
<212>  DNA
<213>  Corynebacterium glutamicum


<220>
<221>  misc_feature
<223>  seqP-RBS_19

<400>  19
tggatacctg aaatcccagt gagcgcaccg ctccccttac gtcacagtct gtaaaacaaa      60

tcttcggtgt tgcgtatcct tgttaataac ttatgcgttg actcattcgt gcacttcggc     120

gtgtcacaat taggtacgac caagaatggg accgggaaac cgggacgtat aaacgaaata     180

aaacattcca acaggaggtg tggaa                                           205


<210>  20
<211>  484
<212>  DNA
<213>  Corynebacterium glutamicum


<220>
<221>  misc_feature
<223>  seqP-RBS_20

<400>  20
ttgaagccta aaaacgaccg agcctattgg gattaccatt gaagccagtg tgagttgcat      60

cacattggct tcaaatctga gactttaatt tgtggattca cgggggtgta atgtagttca     120

taattaaccc cattcggggg agcagatcgt agtgcgaacg atttcaggtt cgttccctgc     180

aaaaactatt tagcgcaagt gttggaaatg cccccgtttg gggtcaatgt ccatttttga     240

atgtgtctgt atgattttgc atctgctgcg aaatctttgt ttccccgcta aagttgagga     300

caggttgaca cggagttgac tcgacgaatt atccaatgtg agtaggtttg gtgcgtgagt     360

tggaaaaatt cgccatactc gcccttgggt tctgtcagct caagaattct tgagtgaccg     420

atgctctgat tgacctaact gcttgacaca ttgcatttcc tacaatcttt agaggagaca     480

caac 484